# EUROPEAN PATENT APPLICATION

(11) **EP 1 779 863 A1**
(43) Date of publication of application: **02.05.2007**
(21) Application number: 07100978.1
(22) Date of filing: 06.10.2000
(51) Int. Cl.: A61K 38/18, A61K 38/30, A23L 1/30, A61P 1/00, A61P 1/04, A61P 1/12, A61K 31/20, A61K 31/715, A61K 38/40, A61K 39/395, A61K 38/05

(54) **Use of TGF-Beta and growth factors in the treatment and prevention of diseases of the intestinal mucosa**

(30) Priority: 06.10.1999 WO PCT/NL99/00620
(62) Divisional of application: 00971890.9
(71) Applicant: N.V. Nutricia, 2712 HM Zoetermeer (NL); Campina, B.V., 5301 LB Zaltbommel (NL)
(72) Inventor: Hoijer, Maarten Anne, 6814 JA, Arnhem (NL); Hageman, Robert Johan Joseph, 6705 CT, Wageningen (NL); Smeets, Rudolf Leonardus Lodewijk, 5912 TD, Venlo (NL)
(74) Representative: Swinkels, Bart Willem

(57) **Abstract**

The invention pertains to the use of transforming growth factor β (TGF-β) in the preparation of a pharmaceutical or nutritional composition for use in the treatment and/or prevention of damage of the intestinal mucosa as a result of chemotherapy or radiotherapy, the pharmaceutical or nutritional composition comprising TGF-β and one or more binding factors for TGF-β in the substantial absence of insulin-like growth factor-1 (IGF-1) which TGF-β is obtainable by extraction from a mammalian milk whey product.

## Description

The present invention relates to a composition containing transforming growth factor β (TGF-β) and its use for the prevention or treatment of malfunction or disease of the intestinal mucosa. The invention further relates to a composition containing TGF-β and specific fibres and/or immunoglobulines and/or calcium which can also be used for such a treatment. The composition containing TGF-β is administered during a period in which it is desired to inhibit cell proliferation and stimulate cell differentiation.

TGF-β is a multifunctional protein found in all mammalian tissues. Currently, five forms of TGF-β are known, β1 to β5. It has been implicated in the development, differentiation and growth of tissue and the control of immune system function and carcinogenesis. TGF-β can be isolated from natural sources (e.g. blood platelets), mammalian milk or colostrum or can be produced by recombinant cells.

IGF-1 is a small protein (molecular weight about 7800) which plays an important role in bone metabolism. It has been shown to stimulate growth of cells in culture. Animal growth is also stimulated in pituitary deficient, normal and catabolic states. Kidney function is also improved. It can be produced using recombinant DNA technology, solid phase peptide synthesis, by isolating it from blood serum or from human or bovine milk.

TGF-β and its uses are for instance described in EP 852913. This document relates to an enteral food preparation which contains casein rich in TGF-β2, a lipid source such as medium chain or long chain triglycerides or polyunsaturated fatty acids and a carbohydrate source, i.e. maltodextrin, corn starch or sucrose. This composition is used in the treatment or prophylaxis of inflammatory conditions of the gastrointestinal tract, such as Crohn's disease.

EP 462,398 describes the combination of TGF-β1 and a polyunsaturated fatty acid (PUFA) such as linoleic acid, alpha-linolenic acid, gamma-linolenic acid, arachidonic acid, dihomo-gamma-linolenic acid, eicosapentaenoic acid and/or docosahexanoic acid and/or a derivative thereof for treatment of neoplastic diseases.

WO 96/34614 describes a method for preventing and/or treating damage to the lining of the alimentary tract resulting from chemotherapy and/or radiation, wherein a milk product extract including a mixture of cell growth factors is administered to a patient. The milk product extract, preferably a cheese whey extract, may contain lactoferrin and lactoperoxidase and it can be supplemented with growth factors such as IGF-1, IGF-2, TGF-β, TGF-α, EGF, PDGF, FGF or KGF. This document does not mention which of the substances mentioned should be present in order to achieve the desired preventing or curing effect.

In an article of S.T. Sonis et al, Cancer Res. 54:1135-1138 (1994); "Prevention of chemotherapy induced ulcerative mucositis by transforming growth factor β3" it is described that TGF-β3 administration reduced proliferation of oral epithelium in vitro and in vivo. Topical application of TGF-β3 to the oral mucosa of the Syrian golden hamster prior to chemotherapy significantly reduced the incidence, severity and duration of oral mucositis, reduced chemotherapy associated weight loss and increased survival. Prevention of mucositis according to this document is based on limiting the rate of basal epithelial cell proliferation by prior administration of a negative growth regulator.

US 5,842,297 describes that TGF-β3 can be administered to slow growth of normal cells and thereby inhibit cytotoxic poisoning of the normal cells in the subject. Typically the TGF-β3 is administered prior to anti-neoplastic therapy. It is further suggested that after neoplastic therapy a mitogen for epithelial cells can be administered. The drawback of the method of this document is that preferably a growth factor obtained by recombinant technology is used. However, these type of growth factors have shown to be less stable during sterilisation treatments. Also the biological availability of these growth factors in the gastrointestinal tract is not yet satisfactory.

It was found according to the invention that for optimum protection of the intestinal mucosa against the damaging effect of chemotherapy and radiotherapy TGF-β should be administered without the presence of IGF-1, in particular any anabolic growth factor, during the chemotherapy or radiotherapy. The use of a TGF-β obtained from extraction from a mammalian milk product appeared to result in better bioavailability of the growth factor and also increased stability. It was furthermore found that after the chemotherapy or radiotherapy, damaging effects that may have occurred during this therapy can be treated by administering anabolic growth factors, in particular IGF-1 in the substantial absence of TGF-β.

The present invention therefore provides the use of TGF-β in the preparation of a product for use in the treatment and/or prevention of malfunction or disease of the intestinal mucosa; the product comprising a pharmaceutical composition comprising TGF-β in the substantial absence of IGF-1, which TGF-β is obtained by extraction from a mammalian milk product.

Preferably, the weight ratio TGF-β/IGF-1 in the pharmaceutical composition is at least 100.

It has been found that when a mixture of growth factors is used, for instance when a milk product extract is used, the beneficial effect of TGF-β is reduced by the presence of anabolic growth factors. Therefore, it is preferred to administer TGF-β in the substantial absence of such growth factors.

According to a preferred embodiment of the invention the pharmaceutical composition comprises TGF-β in substantial absence of AGF. With AGF any anabolic growth factor is meant, i.e any growth factor that would promote cell growth. Examples thereof are: IGF-1, insulin-like growth factor 2 (IGF-2), growth hormone, epidermal growth factor (EGF), transforming growth factor α (TGF-α), mammalian milk growth factor (MMGF = Beta-cellulin) and fibroblast growth factor (FGF). EGF is for instance described in EP 0546 068, MMGF in WO 99/24470. The ratio TGF-β/AGF is preferably at least 50.

According to the present invention when growth factors are mentioned, these include also the active peptide analogues of these growth factors. With peptide analogue is meant any peptide having substantially the same activity as the growth factor, particularly any peptide analogue which is 90% or more homologous with the growth factor.

"Pharmaceutical composition" according to the present invention is meant to include any conventional pharmaceutical preparation such as a capsule, a tablet etc., as well as dietetic preparations such as feed supplements or total feeds.

Chemotherapy and/or radiotherapy are effective at destroying tumours because they target fast-growing tissues. While tumour cells are selectively targeted by anticancer treatments the fast growing tissues of the host are also susceptible, particularly the immune cells of the body and the lining of the alimentary tract. This can result in damage to the linings of the mouth and oesophagus (mucositis, also referred to as stomatitis) and damage to the intestinal lining, commonly in the small bowel and less frequently in the large bowel, leading to severe diarrhoea and pain.

It was found according to the invention that for optimum protection of the intestinal mucosa against the damaging effect of chemotherapy and radiotherapy a composition containing TGF-β should be administered without the presence of IGF-I, preferably any anabolic growth factor, during the chemotherapy or radiotherapy, in particular during at least the period starting at the latest the first day of said chemotherapy or radiotherapy treatment and ending at the latest the last day of treatment. It was furthermore found that after the chemotherapy or radiotherapy, damaging effects that may have occurred during these therapies can be treated by administering a second composition containing AGF, in particular IGF-1, in the substantial absence of TGF-β.

By "damage" is meant any alteration in normal structure or function. Such damage includes mucositis, at least partial loss of mucosal crypt area and/or mucosal villus length, or an increase in bacterial translocation across the alimentary tract.

As the TGF-β to be used according to the present invention, a TGF-β extracted from a mammalian milk product, such as milk or whey, in particular bovine milk or whey, most preferably whey is used. The advantages of such TGF-β are that it comes from a natural source because of the reluctance against products obtained by recombinant techniques, cost effectiveness and the presence of other beneficial components in milk or whey extract, such as immunoglobulins. Milk contains TGF-β1(ca. 15%) and TGF-β2 (ca. 85%). A process for extracting such a TGF-β is described in a copending application of the applicants (PCT/NL/99/00621).

Preferably an extract is used which contains binding factors for TGF-β. This extract can be obtained by using an extraction process, preferably excluding acidic extraction steps (pH < 5.5). Binding factors for TGF-β are defined as components that are able to form a stable complex with TGF-β in aqueous media. In bovine milk these are albumin and Latency Associated Peptide (LAP).

The presence of binding factors results in increased stability of the growth factor, for instance during sterilisation. It also results in better bioavailability of TGF-β and thus in higher activity. The binding factors help survive the growth factors during passage in the intestine, where digestive enzymes may degrade the growth factors resulting in (partial) loss of activity.

One of the important findings of the present inventors was that milk products could be used as a source for growth factors in the treatment of the disorders as mentioned in the present application, but that in order to be useful a separation treatment should be carried out, separating the transforming growth factor from anabolic growth factors.

A TGF-β obtained from bovine whey or milk will in general contain more than 100, preferably more than 700 µg TGF-β per g protein. Such an extract will for instance contain 750 µg TGF-β/g protein. The IGF-1 content in this extract will be less than 4, preferably less than 1 µg/g protein.

Preferably the TGF-β is present in the composition in such an amount that 50 ng to 150 µg per day is administered. In case of a liquid product, this will contain TGF-β in a concentration of 0.5 µg -1.5 mg TGF-β per litre. The patient will be administered about 100 ml per day of such a liquid product.

It is preferred that the pharmaceutical composition according to the invention also contains fibres which upon fermentation form more than 15 g of butyrate per 100 g of short chain fatty acids, preferably more than 20 g of butyrate per 100 g of short chain fatty acids. This characteristic means that the composition should contain fibres that release a relative large amount of butyrate when they are fermented in the intestine (colon). The amount of butyrate can be determined by the method described in Journal of Clinical Nutrition, 1991, no. 53, p. 1418-1424.

Certain disorders of gut function, for instance resulting from chemotherapy can influence the intestinal flora, which causes a temporary decrease of the fermentation to butyrate, in particular in those cases that the patient is also given a large amount of antibiotics. It is therefore important to administer fibres to the patient which stimulate the synthesis of butyrate by bacteria whereby more butyrate is released into the intestine. Butyrate is a preferential energy substrate in certain intestinal cells, and it also inhibits proliferation and increases differentiation of these cells.

If butyrate is administered as its free salt, undesirable off-flavours can occur. Further only part of the butyrate would reach the colon. A sustained release preparation could overcome this problem, however, these preparations are relatively expensive.

Therefore, according to the present invention it is proposed to administer specific fibres which upon fermentation result in butyrate. Such fibres are: resistant starch, oats bran, in particular the arabinoxylan rich fraction that is poor in β-glucan, some soy fibre extracts and wheat bran. Preferably, wheat bran is used. The amount of fibre is such that a daily ratio of 1 to 30 g, preferably 3 to 10 g, is obtained. In a liquid preparation the concentration is thus 10 to 300 g/l.

The TGF-β composition according to the invention preferably contains immunoglobulins, more in particular in combination with the above mentioned fibres. Their main function is to interact with harmful micro-organisms such as bacteria. This prevents the micro-organism from entering the blood circulation system. This situation in particular occurs when the intestinal mucosa of the patient has been damaged as a result of treatment with chemotherapy.

The immunoglobulins can be isolated from milk of mammals which have been hyperimmunised against certain pathogens or they can be isolated from normal bovine whey or milk. With the process described in the above mentioned patent application, using normal cow's milk as a starting material, a preparation is obtained rich in IgG and IgA. 30 to 50 % of the protein fraction consists of immunoglobulins of the type IgG and IgA. The concentration immunoglobulins in the preparation will, in case of a liquid preparation of 100 ml, be 0.1 to 1500 mg/l.

According to a further preferred embodiment the TGF-β composition contains calcium, preferably in combination with the above mentioned fibres, more preferably in combination with said fibres and immunoglobulins. The calcium can be in the form of finely dispersed calcium phosphate, calcium carbonate, calcium citrate or a calcium concentrate from bovine milk. The addition of calcium reduces the risk of infection. Calcium lowers the proliferation rate of the epithelial cells. The amount of calcium is more than 50 mg/100 ml, preferably more than 100 mg/100 ml, for instances 120 mg/100 ml, based on a liquid composition.

It has been found that when a product containing TGF-β, butyrate producing fibres and high levels of calcium salts is administered a synergistic effect occurs resulting in a composition that is effective in preventing damage to epithelial gut cells during chemotherapy and radiotherapy and in the treatment of inflammatory bowel diseases.

Preferably, the pharmaceutical composition containing TGF-β according to the invention also contains one or more of the following ingredients: proteins, fat, minerals, trace elements, vitamins, fatty acids and lactoferrin.

Preferably, proteins are present in an amount of 3 to 10 % protein equivalents, this includes intact protein, peptides and amino acids. The amount of fat is preferably 2 to 10 %, based on the total weight of the preparation. The amount of minerals, trace elements and vitamins is according to the daily recommended dosage.

Preferred vitamins are vitamin A, C and E. Vitamin A and provitamin A are required. Their concentration is preferably more than 130 µgRE/100 ml, in particular more than 300 µg/100 ml. Suitably part of the vitamin A is administered as retinoic acid or a metabolic equivalent thereof. Vitamin C and tocopherols are administered because of their role in the antioxidant cascade. During radiotherapy but also with initial inflammatory reactions they can protect the epithelial cells. The concentration vitamin C or an equivalent thereof is more than 40 mg/100 ml, preferably more than 60 mg/100 ml. The concentration of tocopherols is more than 5 mg, preferably more than 15 mg/100 ml.

The fats should provide sufficient fatty acids. Preferably stearidonic acid (STA) is added. Suitable fatty acids and the amounts and ratios in which they are used are described in PCT/EP98/08409, i.e. the fatty acids gamma-linolenic acid, stearidonic acid and eicosapentaenoic acid together constitute 10 to 500 mg/g of the total amount of fatty acids and gamma-linolenic acid and eicosapentaenoic constitute 20 to 50 wt.% and stearidonic acid forms 15 to 50 wt.% of these three fatty acids.

Lactoferrin can be present because it has anti-bacterial activity against a number of pathogens. This substance can also have a modulating action with initial inflammatory reactions, which are delayed. It is desired to have a daily doses of 0.1 to 3 g of lactoferrin.

It is preferred that the composition contains less than 11 %, preferably less than 6 % digestible carbohydrates. A higher percentage of these substances would affect the taste of the composition. Generally about 4.5 g/100 ml are used. As a source of digestible carbohydrates sucrose, but also slowly digestible carbohydrates can be used.

According to a further embodiment of the invention TGF-β in the substantial absence of insulin-like growth factor 1 IGF-1, in particular in the absence of AGF, and fibres which upon fermentation form more than 15 g of butyrate per 100g of short chain fatty acids and/or immunoglobulins are used for preparing a pharmaceutical composition for treatment and/or prevention of malfunction or disease of the intestinal mucosa, more in particular for treatment and/or prevention of damage of the intestinal mucosa as a result of chemotherapy or radiotherapy or for treatment and/or prevention of inflammatory bowel diseases.

The present invention also relates to a pharmaceutical composition containing TGF-β in the substantial absence of IGF-1, in particular in the absence of AGF, preferably in combination with fibres which upon fermentation form more than 15 g of butyrate per 100 g of short chain fatty acids and/or immunoglobulins.

In case of a liquid composition, such a composition contains per 100 ml
a) 50 ng to 150 µg TGF-β
b) 1 to 30 g fibres
c) 0.01 to 150 mg immunoglobulins
d) 0.03 to 1 g lactoferrin
e) > 50 mg calcium
f) fatty acids
g) > 130 µg RE vitamin A
h) > 40 mg vitamin C
i) > 5 mg tocopherols
j) 3 to 10% protein equivalents

For example, a suitable liquid TGF-β based formula contains per 100 ml:
a) 4 µg TGF-β
b) 5 g wheat bran
c) 2 mg immunoglobulins
d) 0.5 g lactoferrin
e) 80 mg calcium
f) 4 g fat blend containing 30 % MCT, 26 % palm oil, 16 % soy oil, 8 % borage oil, 11 % echium oil, 6.5 % fish oil and 2.5 % egg lipids
g) 300 µg vitamin A
h) 70 mg vitamin C
i) 15 mg α-tocopherol
j) 4 g casein
k) 5 g maltodextrin
Of this formula, 250 ml per day is administered.

The compositions according to the invention can have the form of any oral preparation, for instance capsules, sachets or tablets each containing a predetermined amount of the active ingredient; powders or granules; solutions or suspensions in an aqueous or non-aqueous liquid. Preferred dosage forms are food supplements or total feeds or powders which upon reconstitution with a liquid such as water give a total feed or food supplement. The present invention also relates to tube feeds containing these ingredients.

## Claims

1. Use of transforming growth factor β (TGF-β) in the preparation of a pharmaceutical or nutritional composition for use in the treatment and/or prevention of damage of the intestinal mucosa as a result of chemotherapy or radiotherapy, the pharmaceutical or nutritional composition comprising TGF-β and one or more binding factors for TGF-β in the substantial absence of insulin-like growth factor-1 (IGF-1) which TGF-β is obtainable by extraction from a mammalian milk whey product.

2. Use according to claim 1, wherein said composition comprises TGF-β in substantial absence of AGF.

3. Use according to claim 1 or 2, wherein the weight ratio of TGF-β/IGF-1 is at least 100:1.

4. Use according to any one of the preceding claims, wherein said composition is administered during the period starting at the latest the first day of said chemotherapy or radiotherapy treatment and ending at the latest at the effective end of said treatment.

5. Use according to any one of the preceding claims, wherein said composition contains TGF-β in such an amount that 50 ng to 150 µg TGF-β per day is administered.

6. Use according to any one of the preceding claims, wherein said binding factors comprise albumin or Latency Associated Peptide (LAP), preferably LAP.

7. Use according to any one of the preceding claims, wherein said composition further comprises one or more of :
- fibres which upon fermentation form more than 15 g of butyrate per 100 g of short chain fatty acids;
- immunoglobulins; and
- calcium.

8. Use according to claim 7, wherein said composition contains fibres in such an amount that 1 to 30 g of fibres per day are administered.

9. Use according to claim 8, wherein said fibres comprise resistant starch.

10. Pharmaceutical or nutritional composition comprising transforming growth factor β (TGF-β) and one or more binding factors for TGF-β in substantial absence of insulin-like growth factor-1 (IGF-1), which TGF-β is obtained by extraction from a mammalian milk whey product.

11. Pharmaceutical or nutritional composition according to claim 10, wherein the composition comprises TGF-β in substantial absence of AGF.

12. Pharmaceutical or nutritional composition according to claim 10 or 11, wherein the weight ratio of TGF-β/IGF-1 is at least 100:1.

13. Pharmaceutical or nutritional composition according to any one of claims 10 - 12, wherein said binding factors comprise albumin or Latency Associated Peptide (LAP), preferably LAP.

14. Pharmaceutical or nutritional composition according to any one of claims 10-13, wherein said composition is a liquid and contains 0.5 µg -1.5 mg TGF-β per litre.

15. Pharmaceutical or nutritional composition according to any one of claims 10-14, wherein said composition further comprises one or more of:
- fibres which upon fermentation form more than 15 g of butyrate per 100 g of short chain fatty acids;
- immunoglobulins; and
- calcium.

16. Use of the composition according to claim 15 in the preparation of a pharmaceutical or nutritional composition for use in the treatment and/or prevention of inflammatory bowel diseases.
